Europäisches Patentamt

European Patent Office

Office européen des brevets

(19)

(11) **EP 0 614 878 B1**

(12) **EUROPÄISCHE PATENTSCHRIFT**

(45) Veröffentlichungstag und Bekanntmachung des
Hinweises auf die Patenterteilung:
**23.04.1997 Patentblatt 1997/17**

(51) Int. Cl.$^6$: **C07C 201/04**, C07C 203/00

(21) Anmeldenummer: **94102780.7**

(22) Anmeldetag: **24.02.1994**

(54) **Verfahren zur Herstellung von C1-C4-Alkyl-nitriten**

Process for the preparation of C1-C4 alkyl nitrites

Procédé pour la préparation de nitrites d'alkyle en C1-C4

(84) Benannte Vertragsstaaten:
**BE CH DE ES FR GB IT LI NL**

(30) Priorität: **08.03.1993 DE 4307193**

(43) Veröffentlichungstag der Anmeldung:
**14.09.1994 Patentblatt 1994/37**

(73) Patentinhaber: **BAYER AG**
**51368 Leverkusen (DE)**

(72) Erfinder:
• **Landscheidt, Heinz, Dr.**
**D-47057 Duisburg (DE)**
• **Wagner, Paul, Dr.**
**D-40597 Düsseldorf (DE)**
• **Kricsfalussy, Zoltan, Dr.**
**D-51375 Leverkusen (DE)**
• **Klausener, Alexander, Dr.**
**D-50670 Köln (DE)**

(56) Entgegenhaltungen:
EP-A- 0 046 983           EP-A- 0 076 217
EP-A- 0 310 191           EP-A- 0 425 197

**Beschreibung**

Die vorliegende Erfindung betrifft ein technisches Verfahren zur Herstellung von Alkylnitriten aus Stickstoffoxiden, Sauerstoff und niederen Alkanolen, wobei die Stickstoffoxide mit einem Anteil von mehr als 50 % NO an der Gesamtmenge von Grammatomen N der Stickstoffoxide eingesetzt werden. Das niedere Alkanol wird zu einem Teil im unteren Teil des Reaktors gemeinsam mit den Stickstoffoxiden eingesetzt und zum restlichen Teil im oberen Teil des Reaktors aufgegeben.

Alkylnitrite (Alkylester der salpetrigen Säure) finden vielfältige Verwendung, beispielsweise als Zusatzstoffe für Motoröle, als Stabilisatoren für ungesättigte organische Verbindungen, als Spasmolytika, als Reagenzien für Oximierungen, Nitrosierungen und Diazotierungen sowie als Zwischenprodukte für chemische Synthesen.

Es ist seit langem bekannt, daß man Alkylnitrite, darunter insbesondere Methylnitrit, durch Umsetzung von Stickstoffoxiden mit den korrespondierenden Alkanolen, darunter insbesondere Methanol, unter Beteiligung einer der Menge und Zusammensetzung der eingesetzten Stickstoffoxide angepaßten Menge Sauerstoff herstellen kann. Beispielsweise können bei der Herstellung von Methylnitrit aus Stickstoffoxiden, Sauerstoff und Methanol grundsätzlich die nachfolgend bezeichneten Reaktionen zwischen den beteiligten Komponenten ablaufen:

$$2 NO + O_2 \rightarrow 2 NO_2 \tag{1}$$

$$NO_2 + NO \leftrightarrow N_2O_3 \tag{2}$$

$$R\text{-}OH + N_2O_3 \rightarrow R\text{-}ONO + HNO_2 \ (R = CH_3) \tag{3}$$

$$R\text{-}OH + HNO_2 \rightarrow R\text{-}ONO + H_2O \tag{4}$$

$$N_2O_3 + H_2O \rightarrow 2 HNO_2 \tag{5}$$

$$2 NO_2 \leftrightarrow N_2O_4 \tag{6}$$

$$R\text{-}OH + N_2O_4 \rightarrow R\text{-}ONO + HNO_3 \tag{7}$$

$$N_2O_4 + H_2O \rightarrow HNO_2 + HNO_3 \tag{8}$$

Bei der Herstellung der angestrebten Alkylnitrite geht man bevorzugt so vor, daß möglichst nur die in den Reaktionsgleichungen (1) bis (4) wiedergegebenen Umsetzungen ablaufen und als einziger Abfallstoff Wasser erhalten wird. Die in Reaktionsgleichung (5) wiedergegebene Reaktion ist im allgemeinen nicht vermeidbar, da das zudosierte bzw. nach Reaktionsgleichung (2) gebildete Distickstofftrioxid nicht nur mit dem Alkohol im Sinne der Reaktionsgleichung (3), sondern auch mit dem nach Reaktionsgleichung (4) entstandenen Wasser abreagieren kann. In Gegenwart ausreichender, insbesondere überschüssiger Mengen des Alkohols wird jedoch die dabei entstehende salpetrige Säure im Sinne der Reaktionsgleichung (4) unter Bildung des gewünschten Alkylnitrits und Wassers abgefangen, geht also nicht etwa als Abfallstoff verloren.

Die in den Reaktionsgleichungen (6) bis (8) wiedergegebenen Umsetzungen sind unerwünscht, da sie zur irreversiblen Bildung von Salpetersäure führen. Durch Bildung dieses Nebenproduktes wird die Ausbeute an gewünschtem Alkylnitrit, bezogen auf eingesetztes Stickoxid, verringert. Die entstandene Salpetersäure muß abgetrennt werden, und insbesondere bei Durchführung des Gesamtprozesses in technischem Maßstab fallen dabei Abwässer an, die unter Aufwendung nicht unerheblicher Kosten nachbehandelt werden müssen. Um die nach Reaktionsgleichung (6) ablaufende Dimerisierung des entweder im Gemisch mit Stickstoffmonoxid zudosierten oder über die Gleichung (1) gebildeten Stickstoffdioxids, die die Bildung der Salpetersäure einleitet, weitestgehend auszuschließen, stellt man das stöchiometrische Verhältnis von Stickstoffmonoxid zu Stickstoffdioxid bevorzugt so ein, daß es einen Wert > 1 annimmt, Auf diese Weise gelingt es, die Bildung von Distickstofftetroxid zugunsten der von Distickstofftrioxid zurückzudrängen.

Wie aus den Reaktionsgleichungen (1) bis (3) zu entnehmen ist, kann bei der Einspeisung von Stickoxiden zur Herstellung von Alkylnitriten vorteilhaft beispielsweise folgendermaßen vorgegangen werden:

- Man speist Stickstoffmonoxid und Stickstoffdioxid getrennt oder als Gemisch ein, wobei ein molares Verhältnis $NO:NO_2$ von > 1 vorliegt, was gemäß den obigen Ausführungen gleichfalls die Bildungswahrscheinlichkeit von Distickstofftetroxid verringert und damit verbunden eine Zurückdrängung der Entstehung von Salpetersäure zur Folge hat.

- Man speist zusätzlich Sauerstoff, getrennt von den Stickstoffoxiden oder als Gemisch mit ihnen, ein, und zwar bevorzugt in einem molaren Verhältnis $NO:O_2$ von > 4, bezogen auf die NO-Molzahl, die über die eingesetzte $NO_2$-

Molzahl hinausgeht, was entsprechend den obigen Ausführungen die Bevorzugung der Bildung von Distickstofftrioxid gegenüber der von Distickstofftetroxid und damit verbunden eine Zurückdrängung der Entstehung von Salpetersäure zur Folge hat.

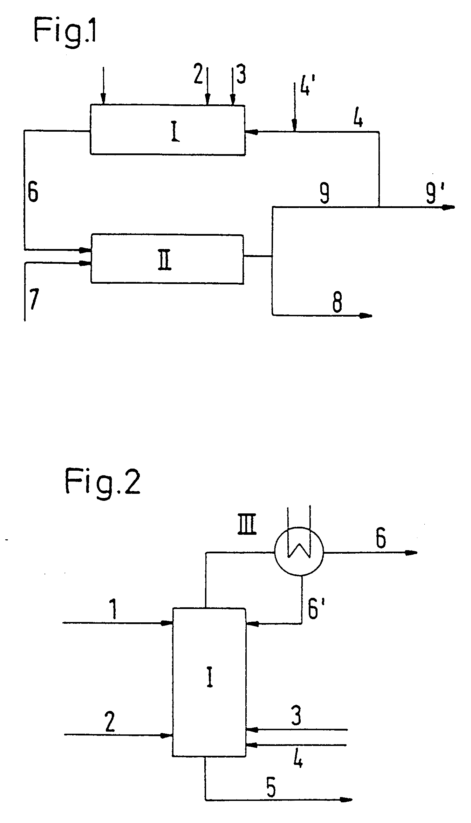

Es sind eine Reihe technisch interessanter kontinuierlich arbeitender Verfahren bekannt, bei denen die Bildung von beispielsweise Methylnitrit in der Gasphase innerhalb eines Gesamtprozesses abläuft, der dadurch gekennzeichnet ist, daß in ihm Methylnitrit als Oxidationsmittel, Cokatalysator, Alkoxylierungsreagenz oder sonstwie gearteter Reaktionspartner fungiert. Typisch für diese Prozesse ist, daß in ihrem Verlauf das im Methylnitrit formal enthaltene Stickstoffmonoxid nicht verbraucht, sondern als Gas freigesetzt wird. In einer bevorzugten Ausführungsform derartiger Reaktionen bringt man das frisch erzeugte Methylnitrit gemeinsam mit dem oder den zusätzlichen Reaktionspartnern und -hilfsstoffen in den Reaktionsraum bzw. das Reaktionsgefäß ein und führt den im gasförmigen Zustand verbleibenden Anteil des Produktgasstromes, der das im Verlaufe der Reaktion gebildete Stickstoffmonoxid enthält, nach weitestgehender Abtrennung des oder der kondensierten bzw. kondensierbaren Reaktionsprodukte in den Reaktor für die Methylnitrit-Herstellung zurück. Damit wird, bezogen auf Stickstoffmonoxid und Methylnitrit, ein Kreisprozeß geschlossen. Das Prinzip eines solchen Prozesses ist in Fig. 1 dargestellt.

Reaktionen, bei denen eine solche kontinuierlich betriebene Kreisfahrweise besonders vorteilhaft ist, sind beispielsweise die folgenden:

(A) Die Herstellung von Oxalsäuredimethylester aus Kohlenmonoxid in Gegenwart geeigneter Katalysatoren [vgl. EP 46 598]

$$2\ CO + 2\ CH_3ONO \xrightarrow[[KAT]]{} H_3COOC\text{-}COOCH_3 + 2\ NO$$

(B) Die Oxidation gegebenenfalls substituierter Olefine in Gegenwart von Methanol und geeigneten Katalysatoren [vgl. EP 55 108]

$$H_2C{=}CH\text{-}R + 2\ CH_3ONO \xrightarrow[[KAT]]{} (H_3CO)_2CH\text{-}CH_2\text{-}R + 2NO$$

(C) Die Herstellung von Dimethylcarbonat aus Kohlenmonoxid in Gegenwart geeigneter Katalysatoren [vgl. EP 425 197 sowie Zeitschrift f. Katalyt. Forschung (China), Vol. 10(1), S. 75-78 (1989)]

$$CO + 2\ CH_3ONO \xrightarrow[[KAT]]{} H_3CO\text{-}OC\text{-}OCH_3 + 2\ NO$$

Die genannten Reaktionen werden in der Gasphase oder auch in der Flüssigphase durchgeführt. In letzterem Falle wird bevorzugt Methanol als Lösungsmittel verwendet. Kennzeichnend ist im allgemeinen, daß sowohl das zugeführte Methylnitrit als auch das entstehende Stickstoffmonoxid gasförmig zu- bzw. abgeleitet werden. Das im Kreis bewegte Gas kann nach Maßgabe der gewählten Reaktionsbedingungen, der Konzentration und der Stoffdaten der beteiligten Komponenten sowie der Phasengleichgewichte über Methylnitrit und/oder Stickstoffmonoxid hinaus noch weitere Substanzen enthalten. Beispielhaft seien Inertgase, wie Kohlendioxid, Stickstoff und Argon, Reaktanden, wie Kohlenmonoxid und Olefine (vgl. (B), z.B. Acrylsäuremethylester), Reaktionsprodukte, wie Oxalsäuredimethylester (vgl. (A)) bzw.Dimethylcarbonat (vgl. (C)) sowie im Gasstrom gelöstes Methanol erwähnt.

Oxalsäuredimethylester ist als Zwischenprodukt von hohem Interesse, beispielsweise für die Herstellung von Ethylenglykol, das seinerseits breite Anwendung als Lösungsmittel, Kühlmittel sowie als Rohstoff für die Fabrikation von Polyesterfasern und Alkydharzen findet.

Acetale, wie die unter (B) beschriebenen, sind wichtige Ausgangsmaterialien für die Herstellung von Heterocyclen, die ihrerseits als Zwischenprodukte bei der Synthese pharmazeutischer und agrochemischer Wirkstoffe verwendet werden.

Dimethylcarbonat findet Anwendung als im Vergleich zu beispielsweise Methylchlorid oder Dimethylsulfat mindertoxisches Methylierungsreagenz, als Treibstoffadditiv sowie als Ausgangsmaterial für die Herstellung von Diphenylcarbonat. Diphenylcarbonat dient als Zwischenprodukt für die Herstellung von Isocyanaten und Polycarbonaten.

Im einzelnen zeigt Fig. 1 einen Reaktor I für die Bildung des Alkylnitrits (beispielsweise des Methylnitrits) und einen Reaktor II für die Durchführung der beschriebenen Reaktionen (A), (B) oder (C) oder einer noch weiteren, Alkylnitrit erfordernden Reaktion. In I gebildetes Alkylnitrit fließt über Leitung (6) nach II, wo es mit weiteren, über (7) zugeführten Ausgangsstoffen umgesetzt wird. Die aus II ablaufenden Stoffe werden in die gewünschten Produkte (8) und in Nebenprodukte (9), die vor allem das erwähnte NO enthalten, getrennt. Die NO enthaltenden Nebenprodukte werden über (4) nach I geführt. Über (9') kann ein Teil der Nebenprodukte als purge stream ausgeschleust werden, um andere Nebenprodukte als das NO nicht über ein vorbestimmtes Niveau ansteigen zu lassen. Die über (9') eintretenden Verluste an NO und anderweitig auftretenden Verluste können über (4') ergänzt werden. Bezugszeichen aus Fig. 1 treten in gleicher Weise auch in Fig. 2 auf, sofern sie die gleiche Bedeutung haben. Dies gilt beispielsweise auch für die Zuführung für Sauerstoff über (3) und Alkanol über (2) und (1).

Wesentlich für das erfolgreiche Betreiben kontinuierlicher Prozesse, wie der in den Beispielen (A), (B) und (C) genannten, ist die Berücksichtigung folgender Forderungen:

- Der zur Regenerierung des Methylnitrits aus dem rückgeführten Stickstoffmonoxid zugespeiste Sauerstoff soll möglichst vollständig zu Methylnitrit umgesetzt werden.

- Dies gilt in gleichem Maße für alle innerhalb des Methylnitrit-Generators befindlichen höheren Stickoxide, namentlich für Stickstoffdioxid, Distickstofftrioxid und Distickstofftetroxid.

- Die Reaktionsführung sowie die Reaktionstechnik sind so zu optimieren, daß die unerwünschte Bildung des Nebenproduktes Salpetersäure (vgl. Reaktionsgleichungen (7) und (8)) weitestgehend ausgeschlossen ist. Dies dient gleichzeitig einer maximal möglichen Ausbeute an gewünschtem Alkylnitrit.

- Die vollständige Umsetzung des zugespeisten Sauerstoffs bzw. der höheren Stickoxide zu dem gewünschten Alkylnitrit soll innerhalb des Alkylnitrit-Generators erfolgen. Damit wird vermieden, daß im Falle einer unvollständigen Abreaktion der beteiligten Komponenten innerhalb des vorgesehenen Reaktionsraumes eine vollständige Umsetzung erst hinter dem Alkylnitrit-Generators eintritt und daß das dabei entstehende Reaktionswasser (vgl. Reaktionsgleichung (4)) in eine etwaige nachgeschaltete Reaktion eingeschleppt wird, was dort gegebenenfalls zu unerwünschten Nebenreaktionen führen kann.

- Das im Verlaufe der Bildung des gewünschten Alkylnitrits entstehende Reaktionswasser sowie die gegebenenfalls infolge unerwünschter Nebenreaktionen gebildete Salpetersäure (vgl. Reaktionsgleichungen (7) und (8)) sollen möglichst vollständig aus dem gasförmigen Produktstrom, der den Alkylnitrit-Generator verläßt, abgetrennt werden.

- Die im Verlaufe der innerhalb des Alkylnitrit-Generators ablaufenden Reaktionen freiwerdende Reaktionswärme ist abzuführen.

- Im Hinblick auf die sicherheitstechnischen Anforderungen an die technische Durchführung von Prozessen, in die Alkylnitrit-Generatoren des hier genannten Prinzips integriert sind, müssen lokale Überhitzungen sowie das Entstehen zündfähiger Gemische vermieden werden.

Alle genannten Anforderungen werden durch das erfindungsgemäße Verfahren auf eine einfache, den Stand der Technik übertreffende Weise erfüllt.

In der Patentanmeldung EP 310 191, die ganz allgemein ein Verfahren für die Herstellung von Alkylnitriten, insbesondere von Methyl- und Ethylnitrit, beschreibt, wird unter Reaktionsbeteiligung von Sauerstoff vorgeschlagen, das gesamte Reaktionsgefäß, in dem die Reaktion zwischen Sauerstoff, Stickstoffmonoxid und dem jeweiligen Alkohol stattfindet, als Wäscher mit zwei räumlich getrennten Zonen, einer Reaktions- und einer Rektifikationszone, auszuführen. Das Waschmedium, in bevorzugter Weise identisch mit dem zur Reaktion gebrachten Alkohol und als solcher im stöchiometrischen Überschuß eingesetzt, wird nach dem Gegenstromprinzip am Kopf der Rektifikationszone eingespeist und dem aufsteigendem Produktgasstrom, bestehend unter anderem aus dem Alkylnitrit und dem bei dessen Bildung entstandenen Wasser, entgegengeführt. Nachteilig ist hierbei die getrennte Auslegung von Reaktions- und Rektifikationszone, die einen hohen apparativen Aufwand erfordert.

Der genannten Patentanmeldung zufolge wird die Rektifikationszone in Form einer Bodenkolonne ausgelegt. Unvorteilhaft bei einer derartigen Vorgehensweise ist, daß oberhalb eines Bodens jeweils eine völlig vermischte Gaszone vorliegt, wobei Rückvermischungseffekte auftreten. Um trotzdem den gewünschten Trenneffekt, d.h. die Entfernung des im Verlaufe der Bildung des Alkylnitrits entstandenen Wassers sowie der wasserlöslichen Nebenprodukte, wie beispielsweise Salpetersäure, zu realisieren, muß der gesamte Apparat deutlich größer dimensioniert werden, als dies bei Ausschluß von Rückvermischungseffekten möglich wäre. Es sei angemerkt, daß beispielsweise etwaige im den Alkylnitritgenerator verlassenden Produktgasstrom enthaltene Salpetersäure oder auch Wasser den nachgeschalteten Prozeß, beispielsweise die Herstellung von Oxalsäuredimethylester oder von Dimethylcarbonat, auch in kleinsten Mengen recht empfindlich stören können.

In der gleichen Patentanmeldung wird beschrieben, daß sich die bei der Entstehung des Alkylnitrits freiwerdende Reaktionswärme dadurch entfernen läßt, daß man aus der Reaktionszone einen flüssigen Seitenstrom abzieht, der nach externer Abkühlung in einen höher gelegenen Teil der Reaktionszone zurückgespeist wird. Dies hat jedoch neben einem erhöhten apparativen Aufwand den weiteren Nachteil, daß dadurch die Konzentration bzw. die Verweilzeit des gebildeten Reaktionswassers innerhalb der Reaktionszone erhöht bzw. verlängert werden. Dabei kann einerseits eine vermehrte Bildung von Nebenprodukten eintreten, andererseits werden der Einsatz gesteigerter Mengen an Waschflüssigkeit, speziell im Falle der Methylnitrit-Herstellung an Methanol, und gegebenenfalls eine weitere Vergrößerung der Rektifikationszone erforderlich, um das am Kopf des gesamten Reaktors austretende Produktgasgemisch weitestgehend wasserfrei zu erhalten.

Schließlich basieren alle Angaben, die der genannten Patentanmeldung EP 310 191 zu entnehmen sind, lediglich auf Computersimulationsrechnungen. Das einzige, gleichfalls auf einer derartigen Computersimulation basierende Beispiel ist für den Fachmann nicht nachvollziehbar, da erstens die ein- und ausgehenden Stoffströme, bezogen auf den Alkylnitritreaktor, nicht spezifiziert werden und zweitens keine Angaben über die Reaktionsvolumina, die allein über die durch die gegebene Reaktionskinetik erforderliche Verweilzeit entscheiden, gemacht werden. Die tatsächliche Effizienz der beschriebenen apparativen Anordnung ist demnach unzureichend belegt und kann daher überhaupt nicht beurteilt werden. Des weiteren ist das genannte Beispiel auch unter technischen Gesichtspunkten irrelevant, da es offenbar keine Inertisierung des grundsätzlich zündfähigen Alkylnitritsstromes beinhaltet und daher die bei sicherheitstechnisch hinreichender Fahrweise auftretende durch den Inertgasanteil bedingte Schleppwirkung keine Berücksichtigung findet. Da sich beispielsweise die untere Explosionsgrenze des Systems Alkylnitrit/Stickstoffmonoxid/Kohlenmonoxid/Alkohol/Inertgas bei steigendem Druck zu kleineren Alkylnitritkonzentrationen hin verschiebt, kommt dieser Frage insbesondere bei Kreisprozessen, wie beispielsweise der technischen Herstellung von Oxalsäuredimethylester oder Dimethylcarbonat, bei denen derartige Gasgemische in Alkylnitrit-Reaktoren eingespeist werden, eine ganz entscheidende Bedeutung zu.

Es bestand daher nach wie vor die Aufgabe, ein Verfahren zur kontinuierlichen Herstellung von $C_1$-$C_4$-Alkyl-nitriten aus den zugrunde liegenden $C_1$-$C_4$-Alkanolen, Sauerstoff und Stickstoffoxiden zu finden, das die obengenannte Forderungen berücksichtigt und sich für die Integration in kontinuierliche Prozesse eignet, bei denen $C_1$-$C_4$-Alkyl-nitrite unter Freisetzung von NO verbraucht werden.

Die Erfindung betrifft ein Verfahren zur Herstellung von $C_1$-$C_4$-Alkylnitriten durch Umsetzung von $C_1$-$C_4$-Alkanolen mit Stickstoffoxiden und Sauerstoff, dadurch gekennzeichnet, daß die Umsetzung in einem als Wäscherkolonne ausgebildeten Reaktor durchgeführt wird, wobei Sauerstoff und Stickstoffoxide in den unteren Teil des Reaktors eingespeist werden, wobei die Stickstoffoxide ein Gemisch eines oder mehrere aus der Gruppe von NO, $N_2O_3$, $NO_2$ und $N_2O_4$ darstellen, von denen NO stets vorliegt und zwar in einer Menge von mehr als 50 % der Gesamtmenge von Grammatomen N der Stickstoffoxide, wobei die Stickstoffoxide mit einem oder mehreren Inertgasen, deren Anteil O-90 Vol-% aller eingesetzten Gase beträgt, vermischt sind, wobei die Sauerstoffmenge 0,15 - 0,3 Mol pro Mol der NO-Molzahl beträgt, die über die $NO_2$-Molzahl hinausgeht, wobei die Alkanolmenge 0,8-2 Mol pro Grammatom N der Stickstoffoxide beträgt, wobei das Alkanol zu 5 - 60 % seiner Gesamtmenge in dampfförmiger oder verdüster Form ebenfalls in den unteren Teil des Reaktors eingespeist wird und das restliche Alkanol im oberen Teil des Reaktors eingespeist wird, wobei bei 10 - 150°C und 0,5 bis 6 bar gearbeitet wird und wobei eine Verweilzeit der Reaktionspartner im Reaktor auf 1 - 500 sec. eingestellt wird.

$C_1$-$C_4$-Alkanole für das erfindungsgemäße Verfahren sind beispielsweise Methanol, Ethanol, Propanol, Isopropanol, Butanol, sek.-Butanol, i-Butanol, bevorzugt Methanol und Ethanol, besonders bevorzugt Methanol. Demzufolge werden die davon abgeleiteten $C_1$-$C_4$-Alkylnitrite, bevorzugt Methylnitrit und Ethylnitrit, besonders bevorzugt Methylnitrit hergestellt.

Das erfindungsgemäße Verfahren arbeitet unter Reaktionsbeteiligung von Sauerstoff.

Es wird erfindungsgemäß angestrebt, als Reaktionspartner des $C_1$-$C_4$-Alkanols Distickstofftrioxid zu bilden, wie es den obigen Gleichungen (3), (4) und (5) entspricht.

Eine wichtige, erfindungsgemäße ablaufende Reaktion ist demnach die Bildung von $N_2O_3$ aus NO und $NO_2$ (Gleichung (2)), wozu $NO_2$ aus NO und $O_2$ gebildet wird (Gleichung (1)).

Daneben kann $N_2O_3$ als solches eingesetzt werden oder als Gemisch mit Stickstoffmonoxid. Schließlich können Gemische Stickstoffmonoxid/Stickstoffdioxid bzw. Stickstoffmonoxid/Stickstofftetroxid, die jeweils zu Distickstofftrioxid reagieren können, eingesetzt werden. Das einzusetzende Gemisch der Stickstoffoxide kann auch alle genannten Spezies, nämlich NO, $N_2O_3$, $NO_2$ und $N_2O_4$ enthalten. In jedem Fall aber liegt NO in einer Menge von mehr als 50 % der Gesamtmenge von Grammatomen N der Stickstoffoxide vor, bevorzugt eine Menge von 51-100 %, besonders bevorzugt 70-100 %, ganz besonders bevorzugt 80-100 %.

Zur gewünschten Bereitstellung von $N_2O_3$ aus NO wird Sauerstoff in einer molaren Menge von 0,15 - 3 Mol, bevorzugt 0,2 - 0,27 Mol, besonders bevorzugt 0,23 bis 0,25 Mol pro Mol der NO-Molzahl eingesetzt, die über die $NO_2$-Molzahl hinausgeht. Etwa vorhandenes $N_2O_4$ wird hierbei als 2 Mol $NO_2$ gezählt. Hierbei muß berücksichtigt werden, daß NO bereits durch Reaktion mit gegebenenfalls vorhandenem $NO_2$ bzw. $N_2O_4$ zu $N_2O_3$ umgesetzt wird.

Die NO-Menge, auf die sich der eingesetzte Sauerstoff in den angegebenen Molverhältnissen bezieht, ist demnach immer die, die in Grammatomen N über die Grammatome N im $NO_2$ und $N_2O_4$ hinausgeht.

Die erfindungsgemäß einzusetzenden Stickstoffoxide oder Stickstoffoxid-Gemische können grundsätzlich ohne Vermischung mit inerten Gasen eingesetzt werden. In bevorzugter Weise wird jedoch ein Inertgas oder ein Gemisch mehrerer von ihnen zugesetzt. Der Anteil des oder der Inertgas(e) beträgt daher 0 bis 90 Vol-% aller eingesetzten Gase, bevorzugt 10 bis 80 Vol-%, besonders bevorzugt 20 bis 70 Vol-%. Als Inertgase kommen hierbei alle in Frage, die mit den Ausgangsstoffen oder den Reaktionsprodukten keine chemischen Reaktionen eingehen. In bevorzugter Weise handelt es sich dabei um eines oder mehrere Gase aus der Gruppe von $CO_2$, $N_2$, Ar, He, bevorzugt aus der Gruppe von $CO_2$ und $N_2$.

Es ist ein wesentliches Kennzeichen des erfindungsgemäßen Verfahrens, daß das umzusetzende $C_1$-$C_4$-Alkanol an zwei Stellen im Reaktor eingesetzt wird. 5 bis 60 % seiner gesamten Einsatzmenge, bevorzugt 10 bis 50 %, besonders bevorzugt 10 bis 30 % des $C_1$-$C_4$-Alkanols werden im unteren Teil des Reaktors in verdampfter oder verdüster Form eingesetzt, während das restliche $C_1$-$C_4$-Alkanol auf den Reaktorkopf gegeben wird. In bevorzugter Weise wird der im unteren Teil des Reaktors eingesetzte Anteil an $C_1$-$C_4$-Alkanol in verdüster Form eingesetzt; der mittlere Tropfendurchmesser beträgt hierbei 5 bis 1.000 μm, bevorzugt 5 bis 500 μm.

Es ist weiterhin eine bevorzugte Variante, die drei Ausgangsstoffströme, die erfindungsgemäß im unteren Teil des Reaktors eingesetzt werden, nämlich das $C_1$-$C_4$-Alkanol, den Sauerstoff und den Stickstoffoxidstrom, gegebenenfalls im Gemisch mit Inertgasen, als einen gemeinsamen Stoffstrom einzusetzen. In einem solchen Fall kann in Kombination mit der bevorzugten Verdüsung des $C_1$-$C_4$-Alkanol-Anteils der Strom der Einsatzgase (Stickstoffoxid, gegebenenfalls mit Inertgas und Sauerstoff) zur Verdüsung des Alkanol-Anteils herangezogen werden.

Die gesamte Einsatzmenge an $C_1$-$C_4$-Alkanol, also sowohl der im unteren Teil des Reaktors als auch im oberen Teil des Reaktors eingesetzte Teil, betragen gemeinsam 0,8 bis 2 Mol je Grammatom Stickstoff im Distickstofftrioxid (bzw. seinen Vorläufer-Gemischen), bevorzugt 1 bis 1,5 Mol, besonders bevorzugt 1 bis 1,2 Mol. Der gesamte Bereich umfaßt also einen geringfügig unterstöchiometrischen Anteil, innerhalb dessen die unerwünschten Nebenreaktionen bis zu einem gewissen Ausmaße toleriert werden, bis zu einem überstöchiometrischen Anteil, bevorzugt jedoch einen nahezu äquimolaren Anteil oder wenig darüber.

Das erfindungsgemäße Verfahren wird bei einer Temperatur von 10 bis 150°C, bevorzugt bei 30 bis 100°C, und bei einem Druck von 0,5 bis 6 bar, bevorzugt 0,8 bis 5 bar, besonders bevorzugt 1 bis 4 bar, durchgeführt, wobei eine Verweilzeit der Reaktionspartner im Reaktor auf 1 bis 500 sec., bevorzugt 5 bis 300 sec., besonders bevorzugt 10 bis 50 sec. eingestellt wird. Temperatur, Druck und Verweilzeit können grundsätzlich unabhängig voneinander eingestellt werden.

Der Reaktor für das erfindungsgemäße Verfahren ist ein als Wäscherkolonne ausgebildeter Reaktor. Solche Kolonnen sind mit Einbauten versehen, die grundsätzlich bekannt sind und für eine innige Flüssig-Gas-Vermischung sorgen. Solche Einbauten sind im wesentlichen die gleichen, die auch bei der Durchführung thermischer Trennoperationen gebräuchlich sind, beispielsweise Böden mit Glocken, Sieblochböden, Ventilböden, Schlitzböden und weitere dem Fachmann bekannte; ferner können Füllkörper aller Art, mit geordneten Packungen, bevorzugt mit strukturierten Packungen, eingesetzt werden; ferner kommen weitere Einbauten wie Umlenkbleche und Schikanebleche in Frage.

Das erfindungsgemäße Verfahren kann beispielhaft mit Hilfe der beigefügten Fig. 2 erläutert werden:

Fig. 2 zeigt einen als Wäscherkolonne ausgebildeten Reaktor I mit einem am Kopf von I angebrachten Kühler III. Das umzusetzende Alkanol wird an den beiden Stellen (1) im oberen Teil und (2) im unteren Teil des Reaktors eingesetzt. Der Alkanolstrom bei (2) wird in verdampfter oder verdüster Form eingesetzt. Bei (3) wird Sauerstoff eingesetzt. Bei (4) werden Stickstoffoxide in der oben beschriebenen Weise, gegebenenfalls vermischt mit Inertgas, eingesetzt. Der Produktgasstrom wird am Kopf des Reaktors entnommen und durch den Kühler III geleitet. In diesem Kühler werden kondensierbare Anteile des Produktstromes, beispielsweise mitgerissenes Alkanol kondensiert und über (6') auf den Kopf der Kolonne, bevorzugt oberhalb der Alkanoleinspeisung (1) zurückgeführt. Der Rest wird über (6) entnommen. Am Sumpf der Kolonne wird Reaktionswasser (5) entnommen. Dieses Reaktionswasser enthält mitentstandene Salpetersäure sowie überschüssig eingesetztes Alkanol.

Zum Verdüsen des Alkanols bei (2) kann Inertgas der oben beschriebenen Art verwendet werden. In der bevorzugten, oben beschriebenen Variante der Zusammenfassung der Einsatzströme (2), (3) und (4) kann der Gasstrom der Stickstoffoxide und des Inertgases sowie des Sauerstoffs zum Verdüsen des Alkanols eingesetzt werden.

In einer bevorzugten Ausführungsform ist das erfindungsgemäße Verfahren Bestandteil eines kontinuierlich arbeitenden Gesamtprozesses, innerhalb dessen $C_1$-$C_4$-Alkylnitrit als Oxidationsmittel, als Cokatalysator, als Alkoxylierungsreagenz oder als sonstwie gearteter Reaktionspartner fungiert, wobei das im Alkylnitrit formal enthaltene Stickstoffmonoxid jedoch nicht für die Herstellung des Produktes verbraucht, sondern gasförmig wieder freigesetzt wird, nach Abtrennung der kondensierten bzw. kondensierbaren Reaktionsprodukte im Produktgasstrom verbleibt und für eine Rückführung in die Alkylnitrit-Synthese zur Verfügung steht. Es liegt somit ein, bezogen auf Stickstoffmonoxid, geschlossener Kreisprozeß vor (vgl. Fig. 1). Der Rückstrom enthält im allgemeinen neben dem genannten Stickstoffmonoxid (0 bis 50 Gew.-%, bevorzugt 5 bis 40 Gew.-%) noch Inertgas, wie beispielsweise Stickstoff, Kohlendioxid, Argon oder Helium (20 bis 80 Gew.-%, bevorzugt 30 bis 70 Gew.-%), gegebenenfalls nicht umgesetztes Alkylnitrit (0 bis 30 Gew.-%, bevorzugt 0 bis 20 Gew.-%) und/oder weitere Komponenten, wie beispielsweise unvollständig umgesetztes Kohlenmonoxid (0 bis 20 Gew.-%), Alkanol, unvollständig auskondensierte Reaktionsprodukte und/oder weitere gasförmige Stoffe. Mögliche Verluste an Stickoxid, die grundsätzlich im Verlaufe der Methylnitrit-Herstellung, der nachgeschalteten Synthese des Reaktionsproduktes, seiner Isolierung, der Rückführung des Kreisgasstromes oder der Auskreisung eines Teilstromes des Kreisgasstromes (purge stream) auftreten können, lassen sich durch Einspeisung von frischem Stickoxid kompensieren. Hierzu lassen sich Stickstoffmonoxid, Stickstoffdioxid, Distickstofftrioxid und Distickstofftetroxid einsetzen. Die Menge des gegebenenfalls zusätzlich einspeisenden Stickoxids beträgt 0 bis 50 Gew.-%, bevorzugt 0 bis 25 Gew.-%, besonders bevorzugt 0 bis 10 Gew.-% des im rückgeführten Kreisgas bereits befindlichen Stickoxids. Die Einspeisung dieses ergänzend zugeführten Stickoxids kann in den Kreisgasstrom hinein erfolgen, beispielsweise unmittelbar vor der Einleitung in den Alkylnitrit-Reaktor. Zusammen mit dem frisch zugeführten Sauerstoff sowie dem Alkanol ergibt sich somit das Reaktionsgemisch für die gewünschte Alkylnitrit-Bildung nach dem erfindungsgemäßen Verfahren. Innerhalb des kontinuierlich arbeitenden Gesamtprozesses ist das ein $C_1$-$C_4$-Alkyl-nitrit erfordernde Verfahren bevorzugt eines der weiter oben als (A), (B) oder (C) geschilderte, besonders bevorzugt das als (C) geschilderte Verfahren der Dialkylcarbonat-Herstellung.

Das erfindungsgemäße Verfahren besitzt verschiedene Vorzüge:

- Die Reaktionsführung sowie die Reaktionstechnik können so optimiert werden, daß die unerwünschte Bildung des Nebenproduktes Salpetersäure weitestgehend ausgeschlossen ist. Dadurch wird gleichzeitig eine maximale Ausbeute an gewünschtem Alkylnitrit erreicht. Überraschenderweise stellt es sich als besonders vorteilhaft für die angestrebte Minimierung der unerwünschten Bildung von Salpetersäure heraus, wenn das eingespeiste Reaktandgas, namentlich das Stickstoffmonoxid und der Sauerstoff, tunlichst unmittelbar nach ihrer Zusammenführung und bei Eintritt in den Reaktor bereits mit einer geeigneten Menge Alkanol unter möglichst schneller und vollständiger Vermischung aller Komponenten in Kontakt gebracht wird. Genau dies wird durch die bevorzugte Verwendung einer Düse für diese Operation, im erfindungsgemäßen Zusammenhang mit der Einspeisung eines Teils des Methanols in den unteren Bereich des Reaktors, bewirkt.

- Die vollständige Umsetzung des eingespeisten Sauerstoffs bzw. der höheren Stickoxide zu dem gewünschten Alkylnitrit erfolgt innerhalb des Alkylnitrit-Generators. Damit wird vermieden, daß im Falle einer unvollständigen Abreaktion der beteiligten Komponenten innerhalb des vorgesehenen Reaktionsraumes eine vollständige Umsetzung erst hinter dem Alkylnitrit-Generators eintritt und daß das dabei entstehende Reaktionswasser in eine etwaige nachgeschaltete Reaktion, bei der das hergestellte Alkylnitrit weiter umgesetzt wird, eingeschleppt wird, wodurch gegebenenfalls unerwünschte Nebenreaktionen verursacht werden können. Sollte es dennoch gewünscht sein, einen unvollständigen Umsatz an Stickoxiden zu erzielen und bespielsweise ein Produktgasgemisch zu erhalten, in dem noch unumgesetztes Stickstoffmonoxid vorhanden ist, so läßt sich dies nach dem erfindungsgemäßen Verfahren gleichfalls realisieren, beispielsweise durch eine Überdosierung des zum Einsatz gebrachten Stickstoffmonoxids.

- Das im Verlaufe der Bildung des gewünschten Alkylnitrits entstehende Reaktionswasser sowie die gegebenenfalls infolge unerwünschter Nebenreaktionen gebildete Salpetersäure werden praktisch vollstandig aus dem gasförmigen Produktstrom, der den Alkylnitrit-Generator verläßt, abgetrennt.

- Die im Verlaufe der innerhalb des Alkylnitrit-Generators ablaufenden Reaktionen freiwerdende Reaktionswärme wird vollständig abgeführt. In überraschender Weise gelingt dies besonders glatt, wenn bei der erfindungsgemäßen und gemeinsam mit der Zuleitung des gasförmigen Reaktanden, namentlich Stickstoffmonoxid und Sauerstoff, erfolgenden Einspeisung eines Teils des Alkanols in den unteren Bereich des Reaktionsgefäßes dieses Methanol in flüssiger Form eingebracht wird (verdüst statt verdampft).

- Im Hinblick auf die sicherheitstechnischen Anforderungen an die technische Durchführung von Prozessen, in die Alkylnitrit-Generatoren des hier genannten Prinzips integriert sind, werden lokale Überhitzungen sowie das Entstehen zündfähiger Gemische vermieden.

- Das erfindungsgemäße Verfahren ist insbesondere für die Integration in kontinuierlich arbeitende Verfahren geeignet, bei denen die Bildung des Alkylnitrits in der Gasphase innerhalb eines Gesamtprozesses abläuft, der dadurch gekennzeichnet ist, daß in ihm Alkylnitrit als Oxidationsmittel, als Cokatalysator, als Alkoxylierungsreagenz oder als sonstwie gearteter Reaktionspartner fungiert, bei dem das im Alkylnitrit formal enthaltene Stickstoffmonoxid nicht für die Herstellung des Produktes verbraucht, sondern als Gas freigesetzt wird und bei dem der nach Abtrennung der kondensierten bzw. kondensierbaren Reaktionsprodukte verbleibende Produktgasstrom und das darin enthaltene Stickstoffmonoxid für die Rückführung in die Alkylnitrit-Synthese zur Verfügung steht, somit also einen, bezogen auf Stickstoffmonoxid und Alkylnitrit, geschlossenen Kreisprozeß darstellt (vgl. Fig. 1).

Beispiele

Beispiel 1

Herstellung von Methylnitrit aus Stickstoffmonoxid, Sauerstoff und Methanol

Es wurde ein Reaktorsystem verwendet, wie es in Fig. 2 schematisch dargestellt ist (Volumen des Reaktors I 16,0 l, 9 theoretische Trennstufen, Kopfdruck 3030 mbar, ausgerüstet mit einem am Kopf des Reaktors angebrachten Wärmeaustauscher III). In der Tabelle sind die zugespeisten bzw. abgeleiteten Ströme mit den aus Fig. 2 entnommenen Ziffern gekennzeichnet. Strom (1) und (5) sind flüssig (fl.), Strom (2) ist, je nach gewählter Verfahrensvariante, flüssig (fl.) oder gasförmig (g.), und Strom (3), (4) und (6) sind gasförmig (g.). Während Strom (1),(2), (3) und (4) die eingesetzten Reaktanden spezifizieren und quantifizieren, sind die sich experimentell ergebenden Gehalte der aufgelisteten Komponenten im Produktgasstrom als Strom (6) und im Ablauf als Strom (5) wiedergegeben.
Im Rahmen der Meßungenauigkeiten enthielten die Ströme folgende Stoffe:

| Strom (1) (fl.) | | Strom (2) | | Strom (3) (g.) | |
|---|---|---|---|---|---|
| MeOH | 2010 g/h | MeOH (g.) | 502 g/h | $O_2$ | 520 g/h |
| Strom 4 (g.) | | Strom 5 (fl.) | | Strom 6 (g.) | |
| NO | 1922 g/h | MeOH | 74 g/h | $CO_2$ | 3373 g/h |
| $CO_2$ | 3373 g/h | $HNO_3$ | 40 g/h | NO | 14 g/h |
| | | Wasser | 560 g/h | MeOH | 422 g/h |
| | | | | MeONO | 3840 g/h |
| | | | | Wasser | <1,5 g/h |
| | | | | $HNO_3$ | 0 g/h |
| MeOH=$CH_3OH$; MeONO=$CH_3ONO$ | | | | | |

Das Produktgasgemisch (Strom (6)) hatte beim Austritt aus dem Wärmeaustauscher III eine Temperatur von 35°C.

Beispiel 2

Herstellung von Methylnitrit aus Stickstoffmonoxid, Stickstoffdioxid, Sauerstoff und Methanol

Versuchsdurchführung und Kennzeichnung der Einzelströme analog Beispiel 1

| Strom (1) (fl.) | | Strom (2) | | Strom (3) (g.) | |
|---|---|---|---|---|---|
| MeOH | 2009 g/h | MeOH (fl.) | 502 g/h | $O_2$ | 172 g/h |
| Strom (4)(g.) | | Strom (5) (fl.) | | Strom (6) (g.) | |
| NO | 1266 g/h | MeOH | 74 g/h | $CO_2$ | 3365 g/h |
| $NO_2$ | 1000 g/h | $HNO_3$ | 40 g/h | NO | 12 g/h |
| $CO_2$ | 3365 g/h | Wasser | 560 g/h | MeOH | 421 g/h |
| | | | | MeONO | 3840 g/h |
| | | | | Wasser | <1,5 g/h |
| | | | | $HNO_3$ | 0 g/h |

Das Produktgasgemisch (Strom (6)) hatte beim Austritt aus dem Wärmeaustauscher III eine Temperatur von 35°C.

Beispiel 3

Herstellung von Methylnitrit durch Umsetzung des nach Abtrennung der kondensierbaren Reaktionsprodukte rückgeführten Kreisgasstromes eines typischen Methylnitrit verbrauchenden und Stickstoffmonoxid produzierenden Prozesses mit Sauerstoff und Methanol

Versuchsdurchführung und Kennzeichnung der Einzelströme analog Beispiel 1

| Strom (1) (fl.) | | Strom (2) | | Strom (3) (g.) | |
|---|---|---|---|---|---|
| MeOH | 1730 g/h | MeOH (fl.) | 401 g/h | $O_2$ | 496 g/h |
| Strom (4) (g.) | | Strom (5) (fl.) | | Strom (6) (g.) | |
| NO | 1845 g/h | MeOH | 84 g/h | $CO_2$ | 4043 g/h |
| MeOH | 362 g/h | $HNO_3$ | 39 g/h | NO | 23 g/h |
| $CO_2$ | 4043 g/h | Wasser | 535 g/h | MeOH | 480 g/h |
| MeONO | 190 g/h | | | MeONO | 3861 g/h |
| | | | | Wasser | <2,0 g/h |
| | | | | $HNO_3$ | 0 g/h |

Das Produktgasgemisch (Strom (6)) hatte beim Austritt aus dem Wärmeaustauscher III eine Temperatur von 35°C.

**Patentansprüche**

1. Verfahren zur Herstellung von $C_1$-$C_4$-Alkylnitriten durch Umsetzung von $C_1$-$C_4$-Alkanolen mit Stickstoffoxiden und Sauerstoff, dadurch gekennzeichnet, daß die Umsetzung in einem als Wäscherkolonne ausgebildeten Reaktor durchgeführt wird, wobei Sauerstoff und Stickstoffoxide in den unteren Teil des Reaktors eingespeist werden, wobei die Stickstoffoxide ein Gemisch eines oder mehrere aus der Gruppe von NO, $N_2O_3$, $NO_2$ und $N_2O_4$ darstellen, von denen NO stets vorliegt und zwar in einer Menge von mehr als 50 % der Gesamtmenge von Grammatomen N der Stickstoffoxide, wobei die Stickstoffoxide mit einem oder mehreren Inertgasen, deren Anteil O-90 Vol-% aller eingesetzten Gase beträgt, vermischt sind, wobei die Sauerstoffmenge 0,15 - 0,3 Mol pro Mol der NO-Molzahl beträgt, die über die $NO_2$-Molzahl hinausgeht, wobei die Alkanolmenge 0,8 bis 2 Mol pro Grammatom N der Stickstoffoxide beträgt, wobei das Alkanol zu 5 - 60 % seiner Gesamtmenge in dampfförmiger oder verdüster Form ebenfalls in den unteren Teil des Reaktors eingespeist wird und das restliche Alkanol im oberen Teil des Reaktors eingespeist wird, wobei bei 10 - 150°C und 0,5 bis 6 bar gearbeitet wird und wobei eine Verweilzeit der Reaktionspartner im Reaktor auf 1 - 500 sec. eingestellt wird.

2. Verfahren nach Anspruch 1, dadurch gekennzeichnet, daß bei einem Druck von 0,8 - 5 bar, bevorzugt 1 bis 4 bar gearbeitet wird und unabhängig vom Druck bei 30 - 100°C gearbeitet wird und unabhängig von Druck und Temperatur eine Verweilzeit von 5 - 300 sec., bevorzugt 10 - 50 sec. eingestellt wird.

3. Verfahren nach Anspruch 1, dadurch gekennzeichnet, daß NO in einer Menge von 51 - 100 %, bevorzugt 70 bis 100 %, besonders bevorzugt 80 - 100 % der Gesamtmenge von Grammatomen N der Stickstoffoxide vorliegt.

4. Verfahren nach Anspruch 1, dadurch gekennzeichnet, daß als Inertgas eines oder mehrere aus der Gruppe von $CO_2$, $N_2$, Ar und He, bevorzugt $CO_2$ und $N_2$, verwendet werden und der Inertgasanteil 10 - 80 Vol-%, bevorzugt 20 - 70 Vol-% aller eingesetzten Gase beträgt.

5. Verfahren nach Anspruch 1, dadurch gekennzeichnet, daß die Alkanolmenge 1 - 1,5 Mol, bevorzugt 1 bis 1,2 Mol pro Grammatom N der Stickstoffoxide beträgt und unabhängig hiervon 10 - 50 %, bevorzugt 10 bis 30 % ihrer Gesamtmenge in den unteren Teil des Reaktors eingespeist werden.

6. Verfahren nach Anspruch 1, dadurch gekennzeichnet, daß 0,2 - 0,27 Mol, bevorzugt 0,23 - 0,25 Mol Sauerstoff pro Mol der NO-Molzahl, die über die $NO_2$-Molzahl hinausgeht, eingesetzt werden.

7. Verfahren nach Anspruch 1, dadurch gekennzeichnet, daß in den unteren Teil des Reaktors ein gemeinsamer Stoffstrom eingespeist wird, der die Stickstoffoxide, den Sauerstoff, das gegebenenfalls vorhandene Inertgas und den in den unteren Teil des Reaktors einzuspeisenden Alkanolanteil enthält.

**8.** Verfahren nach Anspruch 1, dadurch gekennzeichnet, daß der in den unteren Teil des Reaktors eingespeiste Alkanolanteil in verdüster Form eingesetzt wird, wobei der mittlere Tropfendurchmesser 5 bis 1000 µm, bevorzugt 5 - 500 µm beträgt und wobei bevorzugt die Stickstoffoxide, der Sauerstoff und das gegebenenfalls vorhandene Inertgas zur Verdüsung des Alkanolanteils benutzt werden.

**9.** Verfahren nach Anspruch 1, dadurch gekennzeichnet, daß zur Herstellung von $C_1$-$C_2$-Alkylnitrit ein $C_1$-$C_2$-Alkanol umgesetzt und bevorzugt zur Herstellung von Methylnitrit Methanol umgesetzt wird.

**10.** Verfahren nach Anspruch 1, dadurch gekennzeichnet, daß das Gemisch der Stickstoffoxide mit einem mehr als 50 %igen Anteil an NO, bezogen auf die Gesamtmenge von Grammatomen N der Stickstoffoxide, ganz oder teilweise aus einem chemischen Prozeß stammt, in welchem $C_1$-$C_4$-Alkylnitrit eingesetzt wird und als Nebenprodukte Stickstoffoxide, hauptsächlich in Form von NO, gebildet werden.

## Claims

**1.** Process for the preparation of $C_1$-$C_4$-alkyl nitrites by reaction of $C_1$-$C_4$-alkanols with nitrogen oxides and oxygen, characterised in that the reaction is carried out in a reactor designed as a scrubber column, oxygen and nitrogen oxides being fed into the lower part of the reactor, the nitrogen oxides representing a mixture of one or more selected from the group comprising NO, $N_2O_3$, $NO_2$ and $N_2O_4$, of which NO is always present and that is in an amount of more than 50% of the total amount of gram atoms of N of the nitrogen oxides, the nitrogen oxides being mixed with one or more inert gases, the proportion of which is 0-90 % by volume of all gases used, the amount of oxygen being 0.15 - 0.3 mol per mole of the number of moles of NO which exceeds the number of moles of $NO_2$, the amount of alkanol being 0.8 to 2 mol per gram atom of N of the nitrogen oxides, the alkanol being injected at 5 - 60 % of its total amount in vaporous or atomized form like-wise into the lower part of the reactor and the remaining alkanol being fed into the upper part of the reactor, 10 - 150°C and 0.5 to 6 bar being employed and a residence time of the reaction partners in the reactor being set to 1 - 500 sec.

**2.** Process according to Claim 1, characterised in that a pressure of 0.8 - 5 bar, preferably 1 to 4 bar, is employed and, independently of the pressure, 30 - 100°C is employed and, independently of pressure and temperature, a residence time of 5 - 300 sec., preferably 10 - 50 sec., is set.

**3.** Process according to Claim 1, characterised in that NO is present in an amount of 51 - 100%, preferably 70 to 100%, particularly preferably 80 -100% of the total amount of gram atoms of N of the nitrogen oxides.

**4.** Process according to Claim 1, characterised in that the inert gas used is one or more selected from the group comprising $CO_2$, $N_2$, Ar and He, preferably $CO_2$ and $N_2$, and the inert gas proportion is 10 - 80% by volume, preferably 20 - 70% by volume of all gases used.

**5.** Process according to Claim 1, characterised in that the amount of alkanol is 1 - 1.5 mol, preferably 1 to 1.2 mol per gram atom of N of the nitrogen oxides and, independently thereof, 10 - 50%, preferably 10 to 30%, of its overall amount is fed into the lower part of the reactor.

**6.** Process according to Claim 1, characterised in that 0.2 - 0.27 mol, preferably 0.23 - 0.25 mol, of oxygen are used per mole of the number of moles of NO which exceeds the number of moles of $NO_2$.

**7.** Process according to Claim 1, characterised in that, into the lower part of the reactor, is fed a combined mass stream which contains the nitrogen oxides, the oxygen, the inert gas optionally present and the alkanol portion to be fed into the lower part of the reactor.

**8.** Process according to Claim 1, characterised in that the alkanol portion fed into the lower part of the reactor is used in atomized form, the mean droplet diameter being 5 to 1,000 µm, preferably 5 - 500 µm and the nitrogen oxides, the oxygen and the optionally present inert gas being preferably used for atomizing the alkanol portion.

**9.** Process according to Claim 1, characterised in that a $C_1$-$C_2$-alkanol is reacted for the preparation of $C_1$-$C_2$-alkyl nitrite and, preferably, methanol is reacted for the preparation of methyl nitrite.

**10.** Process according to Claim 1, characterised in that the mixture of the nitrogen oxide containing a proportion of NO of more than 50%, based on the total amount of gram atoms of N of the nitrogen oxides, originates in whole or in part from a chemical process in which $C_1$-$C_4$-alkyl nitrite is used and nitrogen oxides, chiefly in the form of NO, are formed as by-products.

## Revendications

**1.** Procédé de préparation de nitrites d'alkyle en $C_1$ à $C_4$ par réaction des alcanols en $C_1$ à $C_4$ avec des oxydes d'azote et l'oxygène, caractérisé en ce que la réaction est exécutée dans un réacteur présentant la forme d'une colonne de lavage, l'oxygène et les oxydes d'azote étant introduits dans la partie inférieure du réacteur, les oxydes d'azote consistant en un mélange d'un ou plusieurs composés choisis parmi NO, $N_2O_3$, $NO_2$ et $N_2O_4$, dans lequel NO est toujours présent, et en fait en quantité supérieure à 50 % de la quantité totale des atomes-grammes de N des oxydes d'azote, ceux-ci étant mélangés avec un ou plusieurs gaz inertes dont la proportion représente de 0 à 90 % en volume de tous les gaz mis en oeuvre, la quantité d'oxygène représentant de 0,15 à 0,3 mole par mole de NO, rapporté au nombre de moles de NO dépassant le nombre de moles de $NO_2$, la quantité d'alcanol représentant de 0,8 à 2 moles par atomes-grammes de N des oxydes d'azote, l'alcanol étant également introduit, pour 5 à 60 % de sa quantité totale, dans la partie inférieure du réacteur, à l'état de vapeurs ou par injection, et le reste de l'alcanol étant introduit dans la partie supérieure du réacteur, les opérations étant réalisées à des températures de 10 à 150°C et des pressions de 0,5 à 6 bars avec une durée de passage des réactifs dans le réacteur qui est réglée à un niveau de 1 à 500 s.

**2.** Procédé selon la revendication 1, caractérisé en ce que l'on opère sous une pression de 0,8 à 5 bars, de préférence de 1 à 4 bars, et que, indépendamment de la pression, on opère à des températures de 30 à 100°C, et que, indépendamment de la pression et de la température, on règle la durée de passage de 5 à 300 s, de préférence de 10 à 50 s.

**3.** Procédé selon la revendication 1, caractérisé en ce NO est présent en quantité de 51 à 100 %, de préférence de 70 à 100 %, et dans les meilleures conditions de 80 à 100 % de la quantité totale des atomes grammes de N des oxydes d'azote.

**4.** Procédé selon la revendication 1, caractérisé en ce que l'on utilise en tant que gaz inertes un ou plusieurs gaz pris dans le groupe formé par $CO_2$, $N_2$, Ar et He, de préférence $CO_2$ et $N_2$, et en ce que la proportion des gaz inertes représente de 10 à 80 %, de préférence de 20 à 70 % du volume de tous les gaz mis en oeuvre.

**5.** Procédé selon la revendication 1, caractérisé en ce que la quantité d'alcanol représente de 1 à 1,5 mole, de préférence de 1 à 1,2 mole par atomes-grammes de N des oxydes d'azote et que, indépendamment de cette quantité, de 10 à 50 %, de préférence de 10 à 30 % de la quantité totale, sont introduits dans la partie inférieure du réacteur.

**6.** Procédé selon la revendication 1, caractérisé en ce que l'on utilise de 0,2 à 0,27 mole, de préférence de 0,23 à 0,25 mole, d'oxygène par mole de NO, rapporté au nombre de moles de NO dépassant le nombre de moles de $NO_2$.

**7.** Procédé selon la revendication 1, caractérisé en ce que l'on introduit dans la partie inférieure du réacteur un courant de matières commun qui contient les oxydes d'azote, l'oxygène, les gaz inertes éventuels et la fraction de l'alcanol à introduire dans la partie inférieure du réacteur.

**8.** Procédé selon la revendication 1, caractérisé en ce que la fraction de l'alcanol introduite dans la partie intérieure du réacteur est injectée, à un diamètre moyen de gouttelette de 5 à 1000 μm, de préférence de 5 à 500 μm, et de préférence en exploitant les oxydes d'azote, l'oxygène et les gaz inertes éventuels pour l'injection de cette fraction d'alcanol.

**9.** Procédé selon la revendication 1, caractérisé en ce que, pour la préparation d'un nitrite d'alkyle en $C_1$ à $C_2$, on convertit un alcanol en $C_1$ à $C_2$, et de préférence, pour la préparation du nitrite de méthyle, le méthanol.

**10.** Procédé selon la revendication 1, caractérisé en ce que le mélange des oxydes d'azote à une proportion supérieure à 50% de NO, par rapport à la quantité totale des atomes-grammes des oxydes d'azote, provient en totalité ou en partie d'une opération chimique dans laquelle on met en oeuvre un nitrite d'alkyle en $C_1$ à $C_4$ et on forme en produits secondaires des oxydes d'azote, principalement sous la forme de NO.

# Fig.1

# Fig.2